# EUROPÄISCHE PATENTSCHRIFT

(11) **EP 1 197 237 B1**
(45) Veröffentlichungstag und Bekanntmachung des Hinweises auf die Patenterteilung: **03.01.2007**
(21) Anmeldenummer: 01120288.4
(22) Anmeldetag: 24.08.2001
(51) Int. Cl.: A61M 16/16

(54) **Vorrichtung zur Beatmung**
Breathing device
Appareil respiratoire

(30) Priorität: 10.10.2000 DE 10049869
(43) Veröffentlichungstag der Anmeldung: 17.04.2002
(73) Patentinhaber: Weinmann Geräte für Medizin GmbH & Co. KG, 22525 Hamburg (DE)
(72) Erfinder: Heinrich, Wolfram, 22523 Hamburg (DE); König, Karl-Heinz, 22850 Norderstedt (DE); Paesch, Rainer, 25451 Quickborn (DE)
(74) Vertreter: Klickow, Hans-Henning

(56) Entgegenhaltungen:
- EP-A- 0 376 584
- EP-A- 0 535 952
- EP-A- 0 885 623
- DE-A- 9 013 145
- DE-A- 19 808 590
- US-A- 4 823 787
- US-A- 4 852 563
- US-A- 5 031 612
- US-A- 5 344 414

## Beschreibung

Die Erfindung betrifft eine Vorrichtung zur Beatmung, die eine Luftversorgung sowie einen Atemluftbefeuchter aufweist, der mit einem Wassertank sowie einer Heizung versehen ist und der an die Luftversorgung ankoppelbar ist sowie bei der die Heizung mindestens ein Heizelement aufweist, das im Bereich des Wassertanks angeordnet ist und bei der der Atemluftbefeuchter über ein Kopplungselement, das mit einem Anschlußelement der Luftversorgung zusammenwirkt, an die Luftversorgung ankoppelbar ist.

Eine typische Verwendung derartiger Atemluftbefeuchter erfolgt im Zusammenhang mit Atemluftversorgungen, die im Rahmen einer CPAP-Therapie eingesetzt werden. Bei einer derartigen CPAP -Therapie (Continious-Positive-Airway-Pressure) wird einem Patienten im Zusammenhang mit der Therapie der Schlafapnoe wenigstens in Phasen eines Auftretens von Krankheitssymptomen Atemgas mit erhöhtem Druck zur Verfügung gestellt, um eine Abstützung des Atemweges zu erreichen.

Zur Vermeidung eines Austrocknens der Atemwege erweist es sich insbesondere bei längeren Beatmungsphasen als zweckmäßig, eine Befeuchtung der Atemluft durchzuführen. Derartige Befeuchtungen der Atemluft können auch bei anderen Anwendungen realisiert werden.

Typischerweise erfolgt eine Beheizung eines Wassertanks des Befeuchtungssystems indirekt über einen unteren metallischen Boden des Wassertanks. Der metallische Boden wird ähnlich wie bei einem Herd auf eine Heizplatte aufgesetzt und im Bereich der Heizplatte sind elektrische Heizelemente angeordnet. Aufgrund der einzuhaltenden hygienischen Anforderungen muß der Boden zum Reinigen abnehmbar sein und trotzdem eine ausreichende Abdichtung gegen das zu erwärmende Wasser gewährleisten.

Durch die indirekte Beheizung des Wassertanks ergeben sich Energieverluste, darüber hinaus muß auch mit einer entsprechend langen Erwärmungszeit gerechnet werden. Da die Atemluftbefeuchter in der Regel im privaten Bereich der Patienten eingesetzt werden, sind zur Vermeidung entsprechend langer Heizzeiten Gegenmaßnahmen der Patienten üblich, beispielsweise das Eingießen von heißem Wasser in den Wassertank. Hieraus resultiert zum einen eine erhöhte Verletzungsgefahr des Patienten durch Hautkontakt mit dem heißem Wasser, darüber hinaus sind die Geräte nicht für eine derartige Betankung vorgesehen, so daß Beschädigungen oder Zerstörungen hervorgerufen werden können.

Aus der US 4,823,787 A ist bereits eine Vorrichtung zur Beatmung bekannt, die eine mit einem Atemluftbefeuchter koppelbare Luftversorgung aufweist. Der Atemluftbefeuchter ist mit einem Wassertank sowie einer Heizung versehen und an die Luftversorgung ankoppelbar.

In der US 5,031,612 wird ein Atemgasbefeuchter beschrieben, unterhalb dessen Bodens ein steuerbares Heizelement angeordnet ist.

Aus der BP-A-0 376 584 ist ein modular aufgebauter Atemluftbefeuchter bekannt, bei dem ein Wassertank über einen beheizbaren Boden temperierbar ist.

In der BP-A-0 535 952 wird ein Atemluftbefeuchter mit einem metallischen Boden beschrieben, der auf einen Gerätesockel als Heizeinrichtung aufsetzbar ist.

In der BP-A-0 885 623 wird ein weiterer zwischen einem Beatmungsschlauch und einer Luftversorgung anordbarer Atemluftbefeuchter beschrieben, der in Abhängigkeit von meßtechnisch erfaßten Parametern regelbar ist.

In der US-A-4 852 563 wird ein Verzweigungselement für eine Beatmungsleitung beschrieben, das über eine Einrastung mit einem Grundelement zusammenfügbar ist. Das Einrastelement greift hierbei mit einer Verzahnung wahlweise in einer Mehrzahl von Radialnuten der miteinander zu verbindenden Bauelemente ein.

Aus der DE 90 13 145 A ist es bekannt, Verbindungselemente zur Kopplung von medizinischen Schläuchen derart zu konstruieren, daß diese bei einem Zusammenfügen ein Klick-Geräusch erzeugen. Ähnliche Verbindungselemente werden auch in der US-A-5,344,414 erwähnt.

Aufgabe der vorliegenden Erfindung ist es, eine Vorrichtung der einleitend genannten Art derart zu konstruieren, daß eine verbesserte Gebrauchstauglichkeit bereitgestellt wird.

Diese Aufgabe wird erfindungsgemäß dadurch gelöst, daß ein Arretierelement zur Fixierung des Atemluftbefeuchters im Bereich der Luftversorgung als eine Einrastkopplung ausgebildet ist und daß das Arretierelement eine bei einem Einrastvorgang ein Klick-Geräusch generierende Gestaltung aufweist, daß ein Wassertank des Atemluftbefeuchters mit einer von einem Verschlußelement abdichtbaren Einfüllöffnung, einem Auslaßelement sowie einem Einlaß versehen ist, daß der Atemluftbefeuchter einen Elektroanschluß aufweist, der mit einem Gegenanschluß der Luftversorgung zusammenkoppelbar ist und daß die Luftversorgung eine Anzeigevorrichtung, Bedienelemente, eine Fördereinrichtung, einen elektrischen Antrieb sowie eine Steuerung für den elektrischen Antrieb aufweist.

Durch die Anordnung des Heizelementes im Bereich des Wassertankes ist es möglich, die thermische Trägheit des Gesamtsystems und damit die erforderliche Heizzeit erheblich zu verkürzen. Darüber hinaus ist es aufgrund der verminderten thermischen Trägheit möglich, verbesserte Steuerungsverfahren einzusetzen, um eine vorgesehene maximale Leistungsaufnahme des Gesamtgerätes zu vermindern. Insbesondere ist es möglich, die elektrische Energieversorgung der Heizung des Atemluftbefeuchters in Abhängigkeit einer jeweiligen aktuellen Leistungsaufnahme des elektrischen Antriebes der Luftversorgung durchzuführen und hierdurch eine Überlagerung von Leistungsaufnahmespitzen zu vermeiden. Durch eine entsprechende Steuerung ist es möglich, eine relativ gleichmäßige Energieaufnahme über die gesamte Betriebszeit zu erreichen und hierdurch die Verwendung eines kleinen und leichten Netzteiles zu unterstützen.

Eine kompakte Ausbildung wird dadurch unterstützt, daß das Heizelement innerhalb des Wassertanks angeordnet ist.

Eine weitere Ausführungsform wird dadurch definiert, daß das Heizelement im Bereich einer Wandung des Wassertanks angeordnet ist.

Eine weitere Verringerung der thermischen Trägheit durch selektive Beheizung lediglich einer aktuell verdunsteten Wassermenge kann dadurch erreicht werden, daß das Heizelement in einem vom Wassertank abgetrennten Erhitzungsraum angeordnet ist.

Eine erhöhte Betriebssicherheit kann dadurch erreicht werden, daß das Heizelement mit einer Temperaturregelung versehen ist.

Insbesondere ist zur Verringerung eines Heizstromes bei zunehmender Temperatur daran gedacht, daß die Temperaturregelung über mindestens ein PTC-Element realisiert ist.

Eine einfache Handhabung wird dadurch unterstützt, daß der Atemluftbefeuchter über ein Kopplungselement, das mit einem Anschlußelement der Luftversorgung zusammenwirkt, an die Luftversorgung ankoppelbar ist.

Eine adaptive Anpassung an aktuelle Betriebsbedingungen kann dadurch erfolgen, daß eine Heizungssteuerung mit einem Temperatursensor zur Erfassung einer Umgebungstemperatur verbunden ist. Ebenfalls ist es möglich, einen Sensor für eine Atemgastemperatur zu verwenden.

Ebenfalls ist daran gedacht, daß eine Heizungssteuerung mit einem Feuchtesensor zur Erfassung einer Umgebungsluftfeuchte verbunden ist.

Eine einfach zu realisierende Steuerung kann dadurch bereitgestellt werden, daß eine Steuerung der Heizung ein Steuerelement zur Vorgabe einer Amplitude eines jeweiligen Heizstromes aufweist.

Eine weitere Vereinfachung durch Verwendung elektronischer Schalter kann dadurch erreicht werden, daß die Steuerung für das Heizelement einen Pulsbreiten-Modulator aufweist.

Eine Vorgabe eines Steuerungs- beziehungsweise Regelungsverhaltens in Abhängigkeit von aktuell vorliegenden Parametern kann dadurch realisiert werden, daß die Steuerung für die Heizung mindestens eine Steuertabelle zur Realisierung einer parameterabhängigen Steuerung aufweist.

Zur Unterstützung einer Gerätesteuerung in Abhängigkeit von einem jeweiligen Zustand des Patienten wird vorgeschlagen, daß sich durch den Atemluftbefeuchter hindurch mindestens bereichsweise eine Druckmeßleitung erstreckt. Der Druckmeßwert kann alternativ oder zusätzlich auch zur Ermittlung der Heizleistung herangezogen werden.

Zu einem kompakten Geräteaufbau sowie einer einfachen Handhabung trägt es ebenfalls bei, daß sich die Druckmeßleitung durch das Kopplungselement hindurch erstreckt.

Eine hohe Gebrauchstauglichkeit wird auch dadurch unterstützt, daß ein Arretierelement zur Fixierung des Atemluftbefeuchters im Bereich der Luftversorgung als eine Einrastkopplung ausgebildet ist.

Eine Signalisierung einer ordnungsgemäßen Zusammenfügung der einzelnen Bauelemente kann dadurch erfolgen, daß das Arretierelement eine bei einem Einrastvorgang ein Klick-Geräusch generierende Gestaltung aufweist.

Eine Energieversorgung für das Heizelement kann dadurch bereitgestellt werden, daß das Heizelement über einen Elektroanschluß mit einer elektrischen Energieversorgung im Bereich der Luftversorgung verbunden ist.

Eine Verwendung offenliegender elektrischer Kontakte kann dadurch vermieden werden, daß das Heizelement induktiv mit einer elektrischen Energieversorgung gekoppelt ist.

Eine typische Anwendung besteht darin, daß der Atemluftbefeuchter zur Ankopplung an eine Luftversorgung ausgebildet ist, die eine Steuerung zur Bereitstellung einer CPAP-Beatmung aufweist.

Eine Variante zur Durchführung des Steuerverfahrens besteht darin, daß eine elektrische Energieversorgung der elektrischen Heizung des Atemluftbefeuchters und des elektrischen Antriebes der Fördereinrichtung zeitlich verschachtelt durchgeführt werden.

Insbesondere ist daran gedacht, daß die zeitliche Verschachtelung durch Anwendung der Pulsbreiten-Modulation durchgeführt wird.

Eine weitere Steuerungsvariante wird dadurch definiert, daß eine elektrische Energieversorgung der Heizung des Atemluftbefeuchters ausschließlich außerhalb von Beschleunigungsphasen der Fördereinrichtung für das Atemgas durchgeführt wird.

In der Zeichnung sind Ausführungsbeispiele der Erfindung schematisch dargestellt. Es zeigen:
- Fig. 1:: Eine perspektivische Darstellung eines Atemluftbefeuchters, der mit einer Luftversorgung verbindbar ist,
- Fig. 2:: eine Seitenansicht des Atemluftbefeuchters gemäß Fig. 1,
- Fig. 3:: eine Vorderansicht gemäß Blickrichtung III in Fig. 2,
- Fig. 4:: eine Draufsicht gemäß Blickrichtung IV in Fig. 2
und
- Fig. 5:: einen Vertikalschnitt gemäß Schnittlinie V-V in Fig. 3.

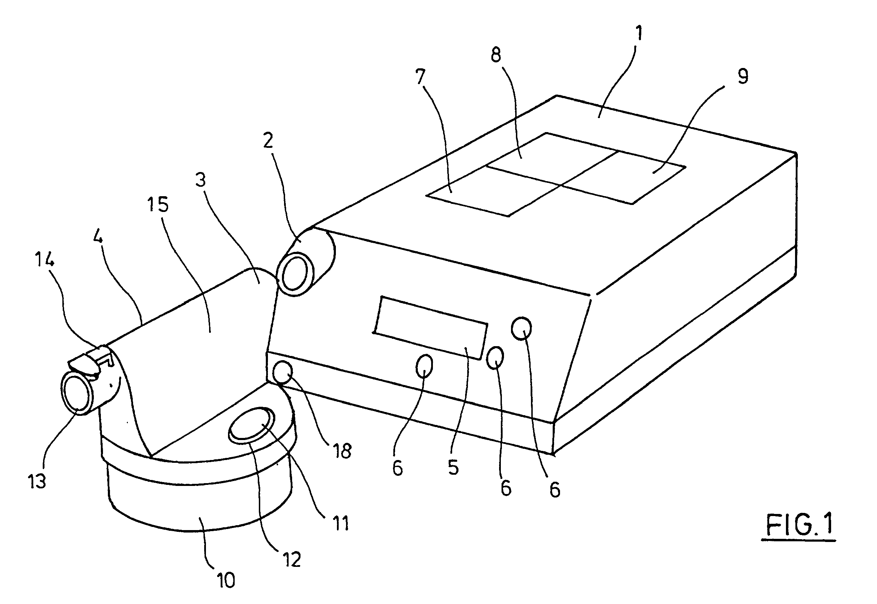

Die perspektivische Darstellung in Fig. 1 zeigt eine Luftversorgung (1), die über ein Anschlußelement (2) mit einem Kopplungselement (3) eines Atemluftbefeuchters (4) verbindbar ist. Das Anschlußelement (2) ist stutzenartig ausgebildet und kann das ebenfalls stutzenförmige Kopplungselement (3) aufnehmen oder bereichsweise von diesem umschlossen werden.

Die Luftversorgung (1) weist eine Anzeigevorrichtung (5) sowie Bedienelemente (6) auf. Die Luftversorgung (1) ist darüber hinaus mit einer Fördereinrichtung (7) für das Atemgas, einem elektrischen Antrieb (8) der Fördereinrichtung (7) sowie einer Steuerung (9) für den elektrischen Antrieb (8) ausgestattet.

Der Atemluftbefeuchter (4) ist mit einem Wassertank (10), einer von einem Verschlußelement (11) abdichtbaren Einfüllöffnung (12) sowie einem Auslaßelement (13) versehen. Das Auslaßelement (13) ist stutzenförmig ausgebildet und dient zum Anschluß eines Beatmungsschlauches. Zur Fixierung des Auslaßelementes (13) im Bereich des Atemluftbefeuchters (4) ist ein Arretierelement (14) vorgesehen, daß in Form eines Klick-Verschlusses ausgebildet sein kann. Oberhalb des Wassertankes (10) erstreckt sich ein Strömungsraum (15).

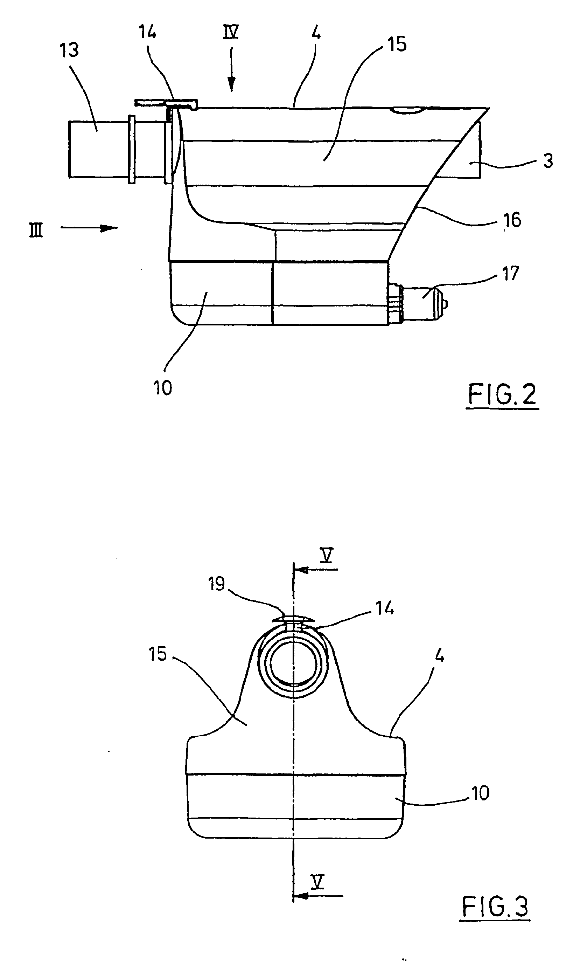

Aus der Seitenansicht in Fig. 2 ist erkennbar, daß eine seitliche Begrenzung (16) des Atemluftbefeuchters (4) einen Konturverlauf aufweist, der an einen Konturverlauf der Luftversorgung (2) im Bereich einer Frontseite angepaßt ist. Hierdurch ist bei einem Zusammenfügen des Atemluftbefeuchter (4) und der Luftversorgung (1) ein sehr kompakter Gesamtaufbau möglich. Darüber hinaus ist aus Fig. 2 erkennbar, daß der Atemluftbefeuchter (4) einen Elektroanschluß (17) aufweist, der mit einem in Fig. 1 dargestellten Gegenanschluß (18) der Luftversorgung (1) zusammenkoppelbar ist.

Aus der Vorderansicht in Fig. 3 ist erkennbar, daß der Atemluftbefeuchter (4) abweichend von Fig. 1 bezüglich einer Mittellinie auch eine im wesentlichen symmetrische Gestaltung besitzen kann. Darüber hinaus ist erkennbar, daß sich das Arretierelement (14) zur Erleichterung einer manuellen Betätigbarkeit mit einem Betätigungsknopf (19) geringfügig über den Außenkonturenverlauf des Atemluftbefeuchters (4) erhebt.

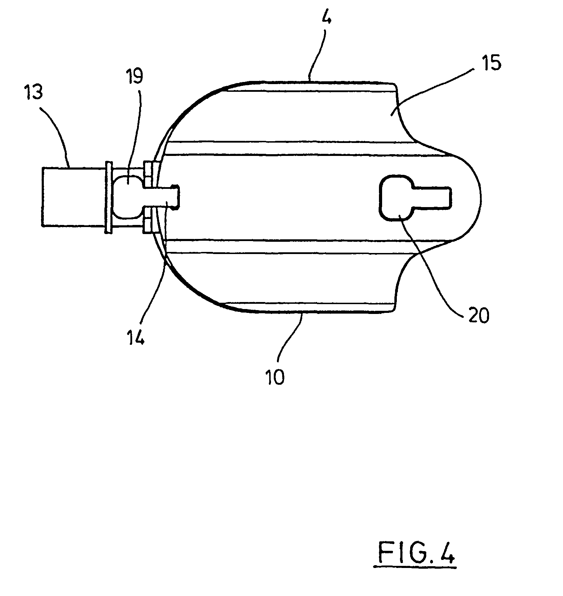

Die Draufsicht in Fig. 4 veranschaulicht, daß der Atemluftbefeuchter (4) mit einem weiteren Arretierelement (20) versehen ist, das eine Arretierung im Verbindungsbereich des Atemluftbefeuchters (4) mit der Luftversorgung (1) durchführt. Das Arretierelement (20) kann im wesentlichen baugleich zum Arretierelement (14) ausgebildet werden.

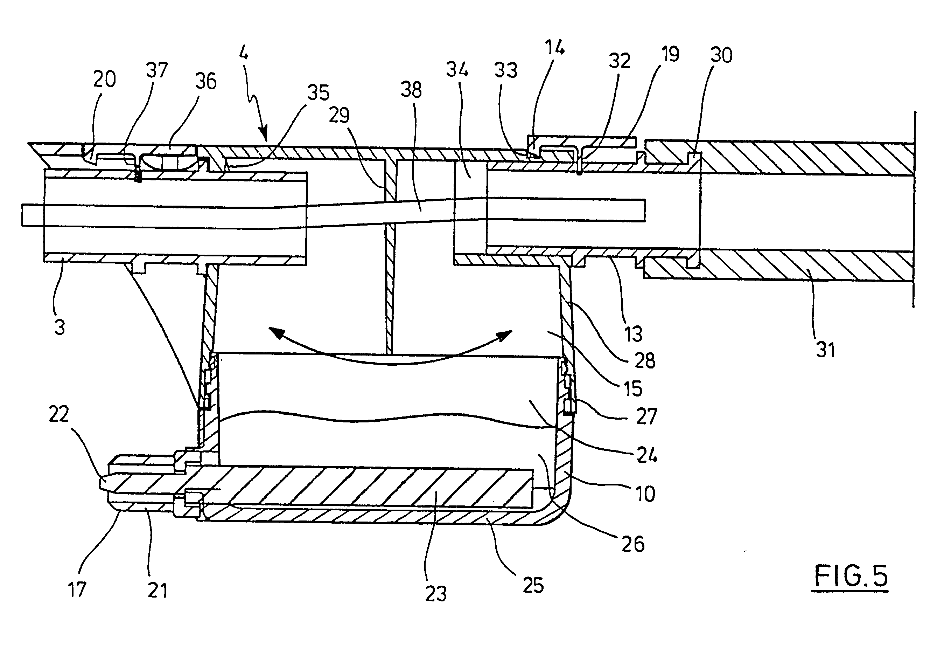

Fig. 5 zeigt in einem Vertikalschnitt den Aufbau des Atemluftbefeuchters (4) im Detail. Es ist erkennbar, daß bei dieser Ausführungsform der Elektroanschluß (17) als ein Koaxialstecker ausgebildet ist, der einen äußeren Ringkontakt (21) sowie einen zentralen Kontaktstift (22) aufweist. Der Elektroanschluß (17) ist mit einem Heizelement (23) verbunden, das bei dieser Ausführungsform im Innenraum (24) des Atemluftbefeuchters (4) oberhalb eines Bodens (25) des Wassertanks (10) angeordnet ist. Das Heizelement (23) wird mindestens bereichsweise direkt von einer Flüssigkeit (26) umspült, die zur Durchführung der Atemluftbefeuchtung innerhalb des Wassertanks (10) angeordnet ist.

Der Vertikalschnitt in Fig. 5 veranschaulicht ebenfalls, daß der Wassertank (10) über einen Verschluß (27) mit einer Haube (28) verbunden ist, die den Strömungsraum (15) umschließt. Der Verschluß (27) kann als ein Bajonettverschluß ausgebildet sein. Zur Unterstützung einer Feuchtigkeitsaufnahme der durch den Strömungsraum (15) hindurchgeleiteten Beatmungsluft ist der Strömungsraum (15) von einer Querwand (29) unterteilt, die einströmende Beatmungsluft in Richtung auf die Flüssigkeit (26) leitet und hierdurch eine Verwirbelung sowie eine erhöhte Feuchtigkeitsaufnahme unterstützt.

Es ist ebenfalls erkennbar, daß das Auslaßelement (13) stutzenartig ausgebildet ist und über ein Kopplungsprofil (30) fest mit einem Beatmungsschlauch (31) verbunden werden kann. Das Arretierelement (14) und der Betätigungsknopf (19) sind über einen Halterungssteg (32) verschwenkbar auf dem Auslaßelement (13) angeordnet, so daß bei einer Druckbeaufschlagung des Betätigungsknopfes (19) das Arretierelement (14) aus einer Arretierprofilierung (33) des Atemluftbefeuchters (4) herausgehoben wird, so daß ein Herausziehen des Auslaßelementes (13) aus einer Führung (34) des Atemluftbefeuchters (4) möglich ist. In umgekehrter Weise erfolgt ein Zusammenfügen des Atemluftbefeuchters (4) und des Auslaßelementes (13) mit dem Beatmungsschlauch (31).

Fig. 5 veranschaulicht ebenfalls, daß das Kopplungselement (3) eine ähnliche Gestaltung wie das Anschlußelement (13) aufweist. Das Kopplungselement (3) ragt durch eine Verbindungsausnehmung (35) in den Innenraum (24) hinein und bei einer Druckbeaufschlagung eines Betätigungsknopfes (36) wird das Arretierelement (20) durch Verschwenkung um einen Halterungssteg (37) herum angehoben beziehungsweise abgesenkt. Hierdurch kann eine Verbindung mit dem Anschlußelement (2) der Luftversorgung (1) hergestellt beziehungsweise wieder gelöst werden.

Zur Unterstützung einer direkten Druckmessung innerhalb des Beatmungsschlauches (31) beziehungsweise des Auslaßelementes (13) erstreckt sich durch den Atemluftbefeuchter (4) eine Druckmeßleitung (38) hindurch, die mit einer zugeordneten Meßwerterfassung im Bereich der Luftversorgung (1) verbunden ist.

Alternativ zu der dargestellten Verbindung des Heizelementes (23) über den Elektroanschluß (17) mit einer im Bereich der Luftversorgung (1) angeordneten Energieversorgung ist es auch möglich, das Heizelement (23) ohne unmittelbaren elektrischen Kontakt, beispielsweise über eine induktive Kopplung, mit einer zugeordneten Energieversorgung zu verbinden.

Das Heizelement (23) kann beispielsweise als eine Heizplatte oder als ein Heizstab realisiert sein. Insbesondere ist daran gedacht, den Wassertank (10) in einen Erhitzungsbereich sowie einen Vorratsbereich zu unterteilen und lediglich eine aktuell zu verdunstende Wassermenge in den Bereich des Heizelementes (23) zu leiten. Dies kann beispielsweise entsprechend dem Prinzip einer Vogeltränke erfolgen. Eine derartige konstruktive Realisierung weist den Vorteil auf, daß lediglich eine geringe Wassermenge für die Verdunstung erwärmt werden zu braucht und daß hierdurch zusätzlich eine Verringerung der thermischen Trägheit erreicht werden. Darüber hinaus werden Energieverluste durch eine Wärmeabstrahlung einer zu großen erwärmten Wassermenge vermieden.

Gemäß einer weiteren Ausführungsform ist vorgesehen, eine Messung eines Stromes durch das Heizelement (23) hindurch vorzunehmen und in Abhängigkeit vom jeweiligen Stromverbrauch eine Steuerung durchzuführen. Insbesondere ist es hierdurch möglich, bei Verwendung von temperaturabhängigen elektrischen Heizwiderständen eine vollständige Verdunstung des Befeuchtungswassers zu erkennen, da bei einer Beendigung des Verdunstungsvorganges eine Temperaturerhöhung des Heizelementes (23) auftritt. Bei einer Detektion eines derartigen Betriebszustandes kann die Heizung abgestellt werden, um eine Beschädigung des Gerätes zu vermeiden.

Durch eine Verwendung von temperaturabhängigen elektrischen Heizwiderständen, beispielsweise von PTC-Heizplättchen, ist es ebenfalls möglich, eine Begrenzung einer jeweiligen Betriebstemperatur auf ein vorgebbares Temperaturniveau vorzunehmen.

Die Steuerung der elektrischen Energieaufnahme des Heizelementes (23) kann durch Pulsweiten-Modulation oder durch die Amplitudenvorgabe eines Gleichstromes oder Wechselstromes erfolgen.

## Patentansprüche

1. Vorrichtung zur Beatmung, die eine Luftversorgung (1) sowie einen Atemluftbefeuchter (4) aufweist, der mit einem wassertank (10) sowie einer Heizung versehen ist und der an die Luftversorgung (1) ankoppelbar ist sowie bei der die Heizung mindestens ein Heizelement (23) aufweist, das im Bereich des Wassertanks (10) angeordnet ist und bei der der Atemluftbefeuchter (4) über ein Kopplungselement (3), das mit einem Anschlußelement (2) der Luftversorgung (1) zusammenwirkt, an die Luftversorgung (1) ankoppelbar ist, **dadurch gekennzeichnet, daß** ein Arretierelement (20) zur Fixierung des Atemluftbefeuchters (4) im Bereich der Luftversorgung (1) als eine Einrastkopplung ausgebildet ist und **daß** das Arretierelement (20) eine bei einem Einrastvorgang ein Klick-Geräusch generierende Gestaltung aufweist, daß ein Wassertank (10) des Atemluftbefeuchters (4) mit einer von einem Verschlußelement (11) abdichtbaren Einfüllöffnung (12), einem Auslaßelement (13) sowie einem Einlaß versehen ist, daß der Atemluftbefeuchter (4) einen Elektroanschluß (17) aufweist, der mit einem Gegenanschluß (18) der Luftversorgung (1) zusammenkoppelbar ist und daß die Luftversorgung (1) eine Anzeigevorrichtung (5), Bedienelemente (6), eine Fördereinrichtung (7), einen elektrischen Antrieb (8) sowie eine Steuerung (9) für den elektrischen Antrieb (8) aufweist.

2. Vorrichtung nach Anspruch 1, **dadurch gekennzeichnet, daß** das Heizelement (23) innerhalb des Wassertanks (10) angeordnet ist.

3. Vorrichtung nach Anspruch 1, **dadurch gekennzeichnet, daß** das Heizelement (23) im Bereich einer Wandung des Wassertanks (10) angeordnet ist.

4. Vorrichtung nach Anspruch 1, **dadurch gekennzeichnet, daß** das Heizelement (23) in einem vom Wassertank (10) abgetrennten Erhitzungsraum angeordnet ist.

5. Vorrichtung nach einem der Ansprüche 1 bis 4, **dadurch gekennzeichnet, daß** das Heizelement (23) mit einer Temperaturregelung versehen ist.

6. Vorrichtung nach einem der Ansprüche 1 bis 5, **dadurch gekennzeichnet, daß** die Temperaturregelung über mindestens ein PTC-Element realisiert ist.

7. Vorrichtung nach einem der Ansprüche 1 bis 6, **dadurch gekennzeichnet, daß** eine Heizungssteuerung mit einem Temperatursensor zur Erfassung einer Umgebungstemperatur verbunden ist.

8. Vorrichtung nach einem der Ansprüche 1 bis 7, **dadurch gekennzeichnet, daß** eine Heizungssteuerung mit einem Temperatursensor zur Erfassung einer Atemgastemperatur verbunden ist.

9. Vorrichtung nach einem der Ansprüche 1 bis 8, **dadurch gekennzeichnet, daß** eine Heizungssteuerung mit einem Feuchtesensor zur Erfassung einer Umgebungsluftfeuchte verbunden ist.

10. Vorrichtung nach einem der Ansprüche 1 bis 9, **dadurch gekennzeichnet, daß** eine Steuerung der Heizung ein Steuerelement zur Vorgabe einer Amplitude eines jeweiligen Heizstromes aufweist.

11. Vorrichtung nach einem der Ansprüche 1 bis 9, **dadurch gekennzeichnet, daß** die Steuerung für das Heizelement einen Pulsbreiten-Modulator aufweist.

12. Vorrichtung nach einem der Ansprüche 1 bis 11, **dadurch gekennzeichnet, daß** die Steuerung für die Heizung mindestens eine Steuertabelle zur Realisierung einer parameterabhängigen Steuerung aufweist.

13. Vorrichtung nach einem der Ansprüche 1 bis 12, **dadurch gekennzeichnet, daß** sich durch den Atemluftbefeuchter (4) hindurch mindestens bereichsweise eine Druckmeßleitung (38) erstreckt.

14. Vorrichtung nach Anspruch 13, **dadurch gekennzeichnet, daß** sich die Druckmeßleitung (38) durch das Kopplungselement (3) hindurch erstreckt.

15. Vorrichtung nach einem der Ansprüche 1 bis 14, **dadurch gekennzeichnet, daß** das Heizelement (23) über einen Elektroanschluß (17) mit einer elektrischen Energieversorgung im Bereich der Luftversorgung (1) verbunden ist.

16. Vorrichtung nach einem der Ansprüche 1 bis 14, **dadurch gekennzeichnet, daß** das Heizelement (23) induktiv mit einer elektrischen Energieversorgung gekoppelt ist.

17. Vorrichtung nach einem der Ansprüche 1 bis 16, **dadurch gekennzeichnet, daß** der Atemluftbefeuchter (4) zur Ankopplung an eine Luftversorgung (1) ausgebildet ist, die eine Steuerung zur Bereitstellung einer CPAP-Beatmung aufweist.

## Claims

1. Breathing apparatus comprising an air supply (1) and a breathing air humidifier (4), which is provided with a water tank (10) and a heating device and can be coupled to the air supply (1), and in which the heating device comprises at least one heating element (23) which is arranged in the region of the water tank (10) and in which the breathing air humidifier (4) can be coupled to the air supply (1) via a coupling element (3) that interacts with a connection element (2) of the air supply (1), **characterised in that** an arresting element (20) for fixing the breathing air humidifier (4) in the region of the air supply (1) is constructed as a engaging coupling, and **in that** the arresting element (20) has a configuration that produces a click in the case of an engaging operation, **in that** a water tank (1) of the breathing air humidifier (4) is provided with a filling aperture (12), which may be sealed by a sealing element (11), an outlet element (13) and an inlet, **in that** the breathing air humidifier (4) comprises an electrical connector (17) which can be coupled to a counter-connector (18) of the air supply (1), and **in that** the air supply (1) comprises a display unit (5), operating elements (6), a conveying device (7), an electrical drive (8) and a controller (9) for the electrical drive (8).

2. Apparatus according to claim 1, **characterised in that** the heating element (23) is arranged inside the water tank (10).

3. Apparatus according to claim 1, **characterised in that** the heating element (23) is arranged in the region of a wall of the water tank (10).

4. Apparatus according to claim 1, **characterised in that** the heating element (23) is arranged in a heating space that is separate from the water tank (10).

5. Apparatus according to any one of claims 1 to 4, **characterised in that** the heating element (23) is provided with a temperature control.

6. Apparatus according to any one of claims 1 to 5, **characterised in that** the temperature control is implemented by way of at least one PTC element.

7. Apparatus according to any one of claims 1 to 6, **characterised in that** a heating controller is connected to a temperature sensor for detecting an ambient temperature.

8. Apparatus according to any one of claims 1 to 7,
**characterised in that** a heating controller is connected to a temperature sensor for detecting a respiratory gas temperature.

9. Apparatus according to any one of claims 1 to 8, **characterised in that** a heating controller is connected to a moisture sensor for detecting environmental moisture.

10. Apparatus according to any one of claims 1 to 9, **characterised in that** a heating device controller comprises a control element for stipulating an amplitude of a respective heating current.

11. Apparatus according to any one of claims 1 to 9, **characterised in that** the controller for the heating element comprises a pulse-width modulator.

12. Apparatus according to any one of claims 1 to 11, **characterised in that** the controller for the heating element comprises at least one control table for achieving parameter-dependent control.

13. Apparatus according to any one of claims 1 to 12, **characterised in that** a pressure measuring line (38) extends, at least in certain sections, through the breathing air humidifier (4).

14. Apparatus according to claim 13, **characterised in that** the pressure measuring line (38) extends through the coupling element (3).

15. Apparatus according to any one of claims 1 to 14, **characterised in that** in the region of the air supply (1) the heating element (23) is connected by an electrical connector (17) to an electrical energy supply.

16. Apparatus according to any one of claims 1 to 14, **characterised in that** the heating element (23) is inductively coupled to an electrical energy supply.

17. Apparatus according to any one of claims 1 to 16, **characterised in that** the breathing air humidifier (4) is constructed for coupling to an air supply (1) which comprises a controller for providing CPAP respiration.

## Revendications

1. Dispositif respiratoire qui présente une alimentation en air (1) ainsi qu'un humidificateur (4) d'air respiratoire doté d'une cuve à eau (10) ainsi que d'un dispositif de chauffage, qui peut être accouplé à l'alimentation en air (1) et qui présente dans le dispositif de chauffage au moins un élément chauffant (23) disposé dans la zone de la cuve à eau (10), l'humidificateur (4) d'air respiratoire pouvant être accouplé à l'alimentation en air (1) par l'intermédiaire d'un élément d'accouplement (3) qui coopère avec un élément de raccordement (2) de l'alimentation en air (1), **caractérisé en ce qu'**un élément d'arrêt (20) qui fixe l'humidificateur (4) d'air respiratoire dans la zone de l'alimentation en air (1) est configuré comme accouplement à encliquetage, **en ce que** l'élément d'arrêt (20) présente une forme qui crée un bruit d'encliquetage lors de l'opération d'encliquetage, **en ce que** la cuve à eau (10) de l'humidificateur (4) d'air respiratoire est dotée d'une ouverture de remplissage (12) qui peut être fermée de manière étanche par un élément de fermeture (11), d'un élément d'évacuation (13) ainsi que d'une admission, **en ce que** l'humidificateur (4) d'air respiratoire présente un raccordement électrique (17) qui peut être accouplé à un raccord complémentaire (18) de l'alimentation en air (1) et **en ce que** l'alimentation en air (1) présente un dispositif d'affichage (5), des éléments de commande (6), un dispositif de transport (7), un entraînement électrique (8) ainsi qu'une commande (9) pour l'entraînement électrique (8).

2. Dispositif selon la revendication 1, **caractérisé en ce que** l'élément chauffant (23) est disposé à l'intérieur de la cuve à eau (10).

3. Dispositif selon la revendication 1, **caractérisé en ce que** l'élément chauffant (23) est disposé dans la zone d'une paroi de la cuve à eau (10).

4. Dispositif selon la revendication 1, **caractérisé en ce que** l'élément chauffant (23) est disposé dans une chambre de chauffage séparée de la cuve à eau (10).

5. Dispositif selon l'une des revendications 1 à 4, **caractérisé en ce que** l'élément chauffant (23) est doté d'une régulation de température.

6. Dispositif selon l'une des revendications 1 à 5, **caractérisé en ce que** la régulation de température est réalisée par au moins un élément CTP.

7. Dispositif selon l'une des revendications 1 à 6, **caractérisé en ce que** la commande du chauffage est reliée à une sonde de température qui détecte la température ambiante.

8. Dispositif selon l'une des revendications 1 à 7, **caractérisé en ce que** la commande du chauffage est reliée à une sonde de température qui détecte la température du gaz respiratoire.

9. Dispositif selon l'une des revendications 1 à 8, **caractérisé en ce que** la commande du chauffage est reliée à un détecteur d'humidité qui détecte l'humidité de l'air ambiant.

10. Dispositif selon l'une des revendications 1 à 9, **caractérisé en ce que** la commande du dispositif de chauffage présente un élément de commande qui définit l'amplitude du courant de chauffage.

11. Dispositif selon l'une des revendications 1 à 9, **caractérisé en ce que** la commande de l'élément chauffant présente un modulateur de largeur d'impulsion.

12. Dispositif selon l'une des revendications 1 à 11, **caractérisé en ce que** la commande du chauffage présente au moins un tableau de commande qui permet de réaliser une commande en fonction de paramètres.

13. Dispositif selon l'une des revendications 1 à 12, **caractérisé en ce qu'**un conduit de mesure de pression (38) s'étend au moins dans certaines parties de l'humidificateur (4) d'air respiratoire.

14. Dispositif selon la revendication 13, **caractérisé en ce que** le conduit (38) de mesure de pression traverse l'élément d'accouplement (3).

15. Dispositif selon l'une des revendications 1 à 14, **caractérisé en ce que** dans la zone de l'alimentation en air (1), l'élément chauffant (23) est relié à une alimentation en énergie électrique par un raccordement électrique (17).

16. Dispositif selon l'une des revendications 1 à 14, **caractérisé en ce que** l'élément chauffant est accouplé par induction à une alimentation en énergie électrique.

17. Dispositif selon l'une des revendications 1 à 16, **caractérisé en ce que** l'humidificateur (4) d'air respiratoire est configuré de manière à pouvoir être accouplé à une alimentation en air (1) qui présente une commande qui permet d'assurer une respiration CPAP.
